# EUROPEAN PATENT SPECIFICATION

(11) **EP 1 285 663 B1**
(45) Date of publication and mention of the grant of the patent: **30.07.2008**
(21) Application number: 01921938.5
(22) Date of filing: 20.04.2001
(51) Int. Cl.: A61K 38/21, A61P 31/00, A61P 1/16, A61P 31/14

(54) **PROTECTIVE INTERFERON-BETA AGAINST REINFECTION AFTER LIVER TRANSPLANTATION**
PROPHYLAKTISCHES INTERFERON-BETA GEGEN NEUERLICHE INFEKTION NACH LEBERTRANSPLANTATION
INTERFERON-BETA COMME AGENT PROTECTEUR CONTRE UNE REINFECTION APRES UNE TRANSPLANTATION HEPATIQUE

(30) Priority: 20.04.2000 JP 2000119174
(43) Date of publication of application: 26.02.2003
(73) Proprietor: TORAY INDUSTRIES, INC., Tokyo 103-8666 (JP)
(72) Inventor: ICHIDA, Takafumi, Niigata-shi, Niigata 950-0088 (JP); KAWASHIMA, Yoshiharu, Yokohama-shi, Kanagawa 227-0052 (JP)
(74) Representative: Kador & Partner
(86) International application number: PCT/JP2001/003385
(87) International publication number: WO 2001/080877

(56) References cited:
- JP-A- 11 171 788
- JP-A- 2000 007 577
- JP-A- 2000 007 578
- SAMUEL D ET AL: "Recurrent hepatitis C after liver transplantation : clinical and therapeutical issues." JOURNAL OF VIRAL HEPATITIS, (2000 MAR) 7 (2) 87-92. REF: 53 JOURNAL CODE: 9435672. ISSN: 1352-0504., March 2000 (2000-03), XP002315838
- CASTRO ANGELES ET AL: "Tolerance and efficacy of subcutaneous interferon-beta administered for treatment of chronic hepatitis C" JOURNAL OF INTERFERON AND CYTOKINE RESEARCH, vol. 17, no. 2, 1997, pages 65-67, XP008007012 ISSN: 1079-9907
- WRIGHT T L ET AL: "Interferon-alpha therapy for hepatitis C virus infection after liver transplantation." HEPATOLOGY (BALTIMORE, MD.) OCT 1994, vol. 20, no. 4 Pt 1, October 1994 (1994-10), pages 773-779, XP008039707 ISSN: 0270-9139
- SAMUEL D ET AL: "HCV infection and liver transplantation ." ACTA GASTRO-ENTEROLOGICA BELGICA, (1997 JUL-SEP) 60 (3) 214-6. REF: 19 JOURNAL CODE: 0414075. ISSN: 0001-5644., 1997, XP008039704
- ZEIN N N: "Interferons in the management of viral hepatitis." CYTOKINES, CELLULAR & MOLECULAR THERAPY, (1998 DEC) 4 (4) 229-41. REF: 151 JOURNAL CODE: 9713367. ISSN: 1368-4736., 1998, XP008039684

## Description

### Technical Field

The present invention relates to a reinfection protecting agent comprising interferon-β as an effective ingredient

### Background Art

A liver disease with the hepatitis C virus(HCV) is a disease which progresses stepwisely from chronic hepatitis due to persistent infection with HCV to cirrhosis, and further progresses to hepatocellular carcinoma(HCC), and liver transplantation is applied to cirrhosis and HCC. However, HCV is known to cause substantially 100% reinfection of a grafted liver after transplantation. Therefore, at present, as a protective measure against reinfection, the amount of the immunosuppressive agent used after transplantation is decreased, or combined therapy using interferon-α and ribavirin is used when a postoperative liver disorder is recognized (Samuel D, Feray C: Recurrent hepatitis C after liver transplantation: clinical and therapeutical issues. J. viral hepatitis 7,87-92 (2000)).

On the other hand, an angiogenetic inhibitory action, a (bFGF) production inhibitory action, and a tumor cell wetting inhibitory action are observed in interferon (referred to as "IFN" hereinafter) (Sidky YA, et al.: Inhibition of Angiogenesis by Interferon: Effect on Tumor- and Lymphocyte-induced Vascular Response. Cancer Res. 47, 5155-5161 (1987)), (Gohji K., et al., Human Recombinant Interferons-Beta and Gamma Decrease Gelatinase Production and Invasion by Human KG-2 Renalcarcinoma Cells. Int. J. Cancer 58, 380-384 (1994)). Also, IFN is known to have a therapeutic effect on the hepatitis C virus (Hiroshi Suzuki, et al.: Treatment of Chronic Active Hepatitis C with Human Interferon-β - a multicenter, randomized, controlled, trial with different duration of administration corresponding to viral load-, KAN · TAN · SUI 38, 571-589, 1999), but a protective effect on reinfection after liver transplantation is not known.

As the protective measure against reinfection after liver transplantation, the above-described method of decreasing the amount of the immunosuppressive agent has an insufficient effect, and reinfection with HCV occurs after liver transplantation, and progresses to chronic hepatitis and cirrhosis, or in some cases, hepatitis progresses to fulminant hepatitis within several years after operation. The combined therapy using IFN-α and ribavirin after operation exhibits an insufficient effective ratio, and there is thus demand for a therapeutic method for sufficient protection against reinfection.

### Disclosure of Invention

The present invention provides a protective agent against reinfection after liver transplantation, which comprises IFN-β as an effective ingredient. Furthermore, the present invention provides a protective method against reinfection after liver transplantation, comprising administering an agent comprising IFN-β as an effective ingredient before liver transplantation.

### Best Mode for Carrying Out the Invention

the present invention relates to a protective agent against reinfection after liver transplantation, comprising IFN-β as an effective ingredient. The IFN-β used in the present invention may be a natural type, a type produced by chemical synthesis, or a type produced by a gene recombination technique. In the present invention, natural IFN-β is preferably used, and natural IFN-β produced by human fibroblasts is more preferably used.

The natural IFN-β can be produced by a method characterized in that a tumorigenic strain and virus are not used. The IFN-β produced in a culture solution is generally a low concentration, and the solution contains many foreign matters derived from cells or additives other than IFN-β. Therefore, the solution is concentrated and purified by a chromatography method using a blue pigment-bound insoluble carrier and a metal chelate group-bound carrier. However, the concentration and purification method is not limited to this.

In the present invention, IFN-β was administered to a patient of HCC complicated with HCV-related cirrhosis before liver transplantation from a living donor. As a result, HCV in the blood continuously disappeared, and reinfection was not recognized. Therefore, the reinfection protecting effect of IFN-β after liver transplantation was confirmed. Based on this result, the protective agent against reinfection can be applied to patients of cirrhosis and HCC caused by HCV ih order to conduct liver transplantation such as liver transplantation from a living donor, liver transplantation from a brain-dead donor, or the like. The agent of the present invention is administered before liver transplantation.

Furthermore, a stabilizer can be added to the protective agent against reinfection of the present invention according to demand. Examples of the stabilizer include human serum albumin, the polyols disclosed in Japanese Unexamined Patent Application Publication No. 58-92619, the organic acid buffers disclosed in Japanese Unexamined Patent Application Publication No. 58-92621,and the like. Furthermore, a carrier in common use may be appropriately mixed with the agent to form a drug product according to the administration method used. Although an injection is used as a dosage form, the dosage form is not limited to this, and various forms such as a capsule agent, a nasal agent, a suppository, a oral agent, an ointment, and the like may be used. The present invention includes use of IFN-β for the manufacture of a protective agent against reinfection after liver transplantation.

In clinical use of the reinfection protecting agent of the present invention, the dosage is appropriately determined according to the object of administration, the administration method, symptoms, etc., but the dosage is preferably in the range of 3,000,000 to 6,000,000 units per day.

In the present invention, the term "HCC" is used because 95% or more of liver cancer in Japan is HCC derived from hepatic cells. As shown by The Japan Society of Hepatology, liver cancer generally represents HCC.

### [Example]

The present invention will be described in further detail below with reference to examples.

A 62-year-old female patient of HCC complicated with HCV-related cirrhosis was intravenously administered with 6,000,000 units of IFN-β for 11 days, and then underwent liver transplantation from a living donor. After transplantation, the amount of the virus in the blood was measured by HCV-RNA quantitative analysis (Amplicore method). HCV in the blood continuously disappeared after transplantation, and reinfection was not recognized (Table 1).

**Table 1**

| | Before administration | | During administration | | The day after administration | Liver transplantation (1 week after administration) | After liver transplantation | | | |
|---|---|---|---|---|---|---|---|---|---|---|
| | 5 months | 3 months | First day | Fifth day | | | 1 month | 2 months | 3 months | 4 months |
| HCV-RNA bDNA probe method (Meq/ml) * | 1.4 | 0.77 | | | | | | | | |
| Amplicore method (copies/ ml)** | | | negative | negative | negative | | negative | negative | negative | negative |

| | | | | | | | | | | |
|---|---|---|---|---|---|---|---|---|---|---|
| * 1 Meq/ml 10⁵ copies/ml (Amplicore method) ** < 10² copies/ml: Negative | | | | | | | | | | |

### Industrial Applicability

The protective effect of IFN-β on virus reinfection after liver transplantation is suggested. This indicates that IFN-β is useful as a reinfection protecting agent.

## Claims

1. Use of interferon-β for manufacturing a protective agent against hepatitis C virus (HCV) reinfection after liver transplantation, comprising administration of interferon-β before liver transplantation.

2. The use according to Claim 1, wherein the liver transplantation is conducted for HCV-related cirrhosis, or HCV-related hepatocellular carcinoma.

3. The use according to Claim 1, wherein the liver transplantation is a liver transplantation from a brain-dead donor.

4. The use according to any one of Claim 1 to 3, wherein the interferon-β is to be administered at 3 million to 6 million units per day prior to the liver transplantation.

## Patentansprüche

1. Verwendung von β-Interferon für die Herstellung eines Mittels zum Schutz vor einer Reinfektion mit dem Hepatitis-C-Virus (HCV) nach einer Lebertransplantation, umfassend die Verabreichung von β-Interferon vor der Lebertransplantation.

2. Verwendung nach Anspruch 1, wobei die Lebertransplantation bei mit dem HCV in Zusammenhang stehender Zirrhose oder einem mit dem HCV in Zusammenhang stehenden, hepatozellulären Karzinom erfolgt.

3. Verwendung nach Anspruch 1, wobei die Lebertransplantation eine Lebertransplantation von einem hirntoten Spender ist.

4. Verwendung nach einem der Ansprüche 1 bis 3, wobei das β-Interferon mit 3 Millionen bis 6 Millionen Einheiten pro Tag vor der Lebertransplantation verabreicht werden soll.

## Revendications

1. Utilisation d'interféron-β pour la préparation d'un agent protecteur vis-à-vis de la réinfection par le virus de l'hépatite VHC après une transplantation du foie, comprenant l'administration d'interféron-β avant la transplantation.

2. Utilisation selon la revendication 1, dans laquelle la transplantation du foie est mise en oeuvre pour une cirrhose liée au VHC ou un carcinome hépato-cellulaire lié au VHC.

3. Utilisation selon la revendication 1, dans laquelle la transplantation du foie est une transplantation du foie provenant d'un donneur en état de mort cérébrale.

4. Utilisation selon l'une quelconque des revendications 1 à 3, dans laquelle l'interféron-β doit être administré à 3 millions à 6 millions d'unités par jour avant la transplantation du foie.
